# EUROPÄISCHE PATENTSCHRIFT

(11) **EP 1 940 874 B1**
(45) Veröffentlichungstag und Bekanntmachung des Hinweises auf die Patenterteilung: **28.12.2011**
(21) Anmeldenummer: 06806613.3
(22) Anmeldetag: 30.10.2006
(51) Int. Cl.: C07K 14/705, C07K 14/33

(54) **DER BOTULINUS NEUROTOXIN A PROTEINREZEPTOR UND SEINE ANWENDUNGEN**
BOTULINUM NEUROTOXIN A PROTEIN RECEPTOR AND USES THEREOF
RECEPTEUR DE PROTEINE DE NEUROTOXINE A DE BOTULINUS ET UTILISATIONS ASSOCIEES

(30) Priorität: 28.10.2005 DE 102005051789
(43) Veröffentlichungstag der Anmeldung: 09.07.2008
(73) Patentinhaber: toxogen GmbH, 30625 Hannover (DE)
(72) Erfinder: RUMMEL, Andreas, 30171 Hannover (DE); BINZ, Thomas, 30539 Hannover (DE); MAHRHOLD, Stefan,Dr., 30659 Hannover (DE); BIGALKE, Johannes Wilhelm, Prof. Dr., 30419 Hannover (DE)
(74) Vertreter: Wilk, Thomas
(86) Internationale Anmeldenummer: PCT/EP2006/010420
(87) Internationale Veröffentlichungsnummer: WO 2007/048638

(56) Entgegenhaltungen:
- WO-A-2005/016233
- JANZ R ET AL: "SV2C is a synaptic vesicle protein with an unsually restricted localization: Anatomy of a synaptic vesicle protein family" NEUROSCIENCE, NEW YORK, NY, US, Bd. 94, Nr. 4, 3. November 1999 (1999-11-03), Seiten 1279-1290, XP002405054 ISSN: 0306-4522
- DATABASE Geneseq [Online] 20. Mai 2003 (2003-05-20), "Rat synaptic vesicle protein 2C." XP002423500 gefunden im EBI accession no. GSP:ABP98503 Database accession no. ABP98503
- DONG MIN ET AL: "SV2 is the protein receptor for botulinum neurotoxin A." SCIENCE 28 APR 2006, Bd. 312, Nr. 5773, 28. April 2006 (2006-04-28), Seiten 592-596, XP002423369 ISSN: 1095-9203
- MAHRHOLD STEFAN ET AL: "The synaptic vesicle protein 2C mediates the uptake of botulinum neurotoxin A into phrenic nerves." FEBS LETTERS 3 APR 2006, Bd. 580, Nr. 8, 3. April 2006 (2006-04-03), Seiten 2011-2014, XP005413387 ISSN: 0014-5793

## Beschreibung

Die vorliegende Erfindung bezieht sich auf ein Polypeptid, das das von *Clostridium botulinum* gebildete Botulinus Neurotoxin A (BoNT/A) bindet. Dieses Polypeptid ist ein Polypeptid der luminale Domäne (Aminosäuren 454-579) des synaptischen Vesikel Glykoproteins 2C (SV2C) aus *Homo sapiens,* das das H_{C}-Fragment von Botulinus Neurotoxin A bindet . Insbesondere bezieht sich die Erfindung auf die Verwendung des Polypeptides und den Peptidabschnitten davon als Antagonist zur Reduktion der Neurotoxizität von BoNT/A, als Hilfsmittel zur Identifikation von Substanzen, welche die Bindung von BoNT/A an Nervenzellen vermindern und zur Detektion von BoNT/A in unterschiedlichen Matrizes.

Nervenzellen setzen Transmitterstoffe durch Exozytose frei. Als Exozytose wird die Verschmelzung der Membranen intrazellulärer Vesikel mit der Plasmamembran bezeichnet. Bei diesem Vorgang wird gleichzeitig der vesikuläre Inhalt in den synaptischen Spalt ausgeschüttet. Die Fusion der beiden Membranen wird durch Calcium reguliert, welches mit dem Protein Synaptotagmin reagiert. Zusammen mit anderen Kofaktoren kontrolliert Synaptotagmin den Status von drei so genannten Fusionsproteinen, dem SNAP-25, Synaptobrevin 2 und Syntaxin 1A. Während Syntaxin 1A und Synaptobrevin 2 in die Plasma- bzw. Vesikelmembran integriert sind, ist SNAP-25 nur schwach an die Plasmamembran gebunden. Bei einem Anstieg der intrazellulären Calcium-Konzentration binden die drei Proteine aneinander, wobei sich beide Membranen annähern und anschliesend miteinander verschmelzen. Bei cholinergen Neuronen wird Acetylcholin freigesetzt, welches Muskelkontraktionen, Schweißabsonderung und andere cholinerg vermittelte Reaktionen verursacht.

Die oben genannten Fusionsproteine sind die Zielmoleküle (Substrate) der leichten Kette (LC) der clostridiellen Neurotoxine, die von den Bakterien *C. botulinum, C. butyricum, C. baratii* und *C*. *tetani* gebildet werden.

Das anaerobe, gram-positive Bakterium *C*. *botulinum* produziert sieben verschiedene Serotypen der clostridiellen Neurotoxine. Diese werden als die Botulinus Neurotoxine (BoNT/A bis BoNT/G) bezeichnet. Hiervon verursachen insbesondere BoNT/A und BoNT/B bei Mensch und Tier eine neuroparalytische Erkrankung, die als Botulismus bezeichnet wird. Die Sporen von *C*. *botulinum* finden sich im Erdreich, können sich jedoch in unsachgemäß sterilisierten und verschlossenen Nahrungsmittelkonserven aus häuslicher Herstellung entwickeln, auf die viele der Botulismusfälle zurückgeführt werden.

BoNT/A ist die aktivste aller bekannten biologischen Substanzen. Nur etwa 5-6 pg gereinigtes BoNT/A repräsentieren eine MLD (Minimale Ietale Dosis). Eine Einheit (engl.: Unit, U) von BoNT/A wird als die MLD definiert, die nach intraperitonealer Injektion die, Hälfte an weiblichen Swiss Webster-Mäusen im einem Gewicht von jeweils 18-20 g tötet. Sieben immunologisch unterschiedliche BoNT wurden charakterisiert. Sie tragen die Bezeichnungen BoNT/A, B, C1, D, E, F und G und können durch Neutralisation mit Serotyp spezifischen Antikörpern unterschieden werden. Die verschiedenen Serotypen von BoNT unterscheiden sich bei betroffenen Tierarten hinsichtlich der Schwere und Dauer der verursachten Lähmung. So ist z. B. bei der Ratte im Hinblick auf die Lähmung BoNT/A 500 mal stärker wirksam als BoNT/B. Hinzu kommt, dass BoNT/B sich bei Primaten in einer Dosierung von 480 U/kg Körpergewicht als untoxisch erwiesen hat. Dieselbe Menge BoNT/A entspricht der 12fachen letalen Dosis dieses Stoffes bei Primaten. Zum anderen ist bei Mäusen die Lähmungsdauer nach Injektion von BbNT/A 10fach länger als nach Injektion von BoNT/E.

Die BoNT werden zur Behandlung neuromuskulärer Störungen eingesetzt, die durch Hyperaktivität in Skelettmuskeln, verursacht durch pathologisch überaktive periphere Nerven, charakterisiert sind. BoNT/A ist von der U.S. Food and Drug Administration zur Behandlung von Blepharospasmus, Strabismus, Hyperhydrose, Falten und Hemifacialspasmen zugelassen. Verglichen mit BoNT/A besitzen die übrigen BoNT Serotypen offensichtlich eine geringere Wirksamkeit und kürzere Wirkdauer. Klinische Effekte des peripher intramuskulär verabreichten BoNT/A stellen sich für gewöhnlich innerhalb einer Woche ein. Die Dauer der Symptomunterdrücküng durch eine einzige intramuskuläre Injektion von BoNT/A beläuft sich im Regelfall auf etwa drei bis sechs Monate.

Die clostridiellen Neurotoxine hydrolysieren spezifisch verschiedene Proteine des Fusionsapparates. BoNT/A, C1 und E spalten SNAP-25, während BoNT/B, D, F, G sowie Tetanus Neurotoxin (TeNT) das vesikel-assoziierte Membranprotein (VAMP) 2 - auch Synaptobrevin 2 genannt - angreifen. BoNT/Cl spaltet außerdem Syntaxin 1A .

Die Clostridien Bakterien setzen die Neurotoxine als einkettige Polypeptide mit jeweils 1251 bis 1315 Aminosäuren frei. Nachfolgend spalten endogene Proteasen jedes dieser Proteine an einer bestimmten Stelle in jeweils 2 Ketten (,nicking'), wobei die beiden Ketten jedoch durch eine Disulfid-Brücke miteinander verbunden bleiben. Diese zweikettigen Proteine werden als Holotoxine bezeichnet (siehe Shone et al. (1985), Eur. J. Biochem. 151, 75-82). Die beiden Ketten haben verschiedene Funktionen. Während das kleinere Teilstück, die leichte Kette (light chain = LC), eine Zn²⁺ -abhängige Endoprotease darstellt, ist die größere Einheit (heavy chain = HC) der Transporter der leichten Kette. Durch Behandlung der HC mit Endopeptidasen ergaben sich zwei 50 kDa Fragmente (siehe Gimenez et al. (1993), J. Protein Chem. 12, 351-363). Die amino-terminale Hälfte (H_{N}-Fragment) integriert sich bei niedrigem pH-Wert in Membranen und transloziert die LC in das Zytosol der Nervenzelle. Die carboxyl-terminale Hälfte (H_{C}-Fragment) bindet an komplexe Polysialoganolioside, die nur in Nervenzellmembranen vorkommen, und an bislang nur teilweise identifizierte Proteinrezeptoren. Dies erklärt die hohe Neuroselektivität der clostridiellen Neurotoxine. Kristallstrukturen bestätigen, dass BoNT/A über drei Domänen verfügt, die mit den drei Schritten des Wirkmechanismus in Einklang gebracht werden können (siehe Lacy et al. (1998), Nat. Struct. Biol. 5, 898-902). Desweiteren lassen diese Daten darauf schließen, dass innerhalb des H_{C}-Fragments zwei autonome Untereinheiten (Subdomänen) von jeweils 25 kDa existieren. Der erste Beweis für die Existenz der beiden funktionellen Subdomänen wurde mit der amino-terminalen (H_{CN}) und der carboxyl-terminalen Hälfte (H_{CC}) des H_{C}-Fragments des TeNT erbracht, die rekombinant exprimiert wurden und erkennen ließen, dass zwar die H_{CC}-, nicht aber die H_{CN}-Domäne an Neurone bindet (siehe Herreros et al. (2000), Biochem. J. 347, 199-204). Zu einem späteren Zeitpunkt wurde eine einzige Gangliosid-Bindungsstelle innerhalb der H_{CC}-Domänen von BoNT/A und B lokalisiert und charakterisiert (siehe Rummel et al. (2004), Mol. Microbiol. 51, 631-643). Der Ort für die Bindung des als Proteinrezeptor für BoNT/B und G identifizierten Synaptotagmins **I** und **II** konnte gleichfalls auf den Bereich der H_{CC}-Domänen von BoNT/B und G beschränkt werden (siehe Rummel et al. (2004), J. Biol. Chem. 279, 30865-70). BoNT/A zeigt weder in PC12 Zellen noch in *in vitro* Proteinbindungsstudien eine irgendwie geartete Interaktion mit einem der zurzeit 13 Mitglieder der Synaptotagmin Proteinfamilie.

Der vorliegenden Erfindung liegt daher die Aufgabe zu Grunde, Mittel und Verfahren zur Beeinflussung der Neurotoxizität von BoNT/A bereitzustellen.

Die Aufgabe wird gelöst durch die Bereitstellung eines Polypeptides der luminalen Domäne (Aminosäuren 454-579) des synaptischen Vesikel Glykoproteins 2C aus *Homo sapiens*, das das H_{C}-Fragment von Botulinus Neurotoxin A bindet . Die vorliegende Erfindung bezieht sich ferner auf die Verwendung des Polypeptides als Antagonist zur Reduktion der Neurotoxizität von BoNT/A, als Hilfsmittel zur Identifikation von Substanzen, welche die Bindung von BoNT/A an Nervenzellen vennindern und zum Nachweis von BoNT/A in unterschiedlichen Matrizes.

Mit der vorliegenden Erfindung wird nun synaptisches Vesikelprotein 2C (SV2C) als Rezeptor für BoNT/A vorgestellt.

In Studien konnte der Erfinder zeigen, dass weder Synaptophysin, Synaptoporin, Synaptogyrin I & III, synaptisches Vesikel Glykoprotein 2A (SV2A) noch synaptisches Vesikel Glykoprotein 2B (SV2B) als Proteinrezeptor für BoNT/A fungieren. Allerdings konnte eine Bindung von BoNT/A an SV2C gezeigt werden.

In einer Ligand-Rezeptor-Studie wurden die luminalen Domänen der Proteine Synaptophysin, Synaptoporin, Synaptogyrin I & III, SV2A, SV2B und SV2C subkloniert und als Gluthathion-S-Transferase-(GST)-Fusionsprotein rekombiant in *E. coli* exprimiert und isoliert. Die H_{C}-Fragmente der sieben BoNTs und von TeNT wurden sowohl rekombinant in *E*. *coli* exprimiert als auch mit ³⁵S-Methionin *in vitro* translatiert. Die Affinität der H_{C}-Fragmente an die luminalen Domänen der oben aufgeführten GST-Fusionsproteine wurde in Glutathion-S-Transferase-(GST)-Pull-down Experimenten bestimmt. In Anwesenheit von GST-SV2C Derivaten wurde die Inhibition der Neurotoxizität von BoNT/A und BoNT/B am isolierten Nerv-Muskel-Präparat der Maus (Hemi-Diaphragma-Assay = HDA), welches das physiologische Ziel der clostridiellen Neurotoxine darstellt, analysiert.

Insbesondere die luminale Domäne von SV2C (Aminosäuren 454-579) stellt das Fragment zur Interaktion mit BoNT/A dar. Das isolierte 125mer Peptid der luminälen Domäne kann ohne Gangliosid mit dem H_{C}-Fragment von BoNT/A interagieren. Durch die Interaktion des SV2C Peptids mit BoNT/A wird dessen Rezeptorbindungsstelle belegt und die Wechselwirkung mit in der Membran eingelagerten SV2C blockiert. Im Detail beinhaltet die vorliegende Erfindung ein 125mer Peptid, das die luminale Domäne von SV2C umfasst . . Diese Agenzien können eingesetzt werden zur spezifischen Bindung an das H_{C}-Fragment von BoNT/A. Dadurch wird die Rezeptorbindungsstelle von BoNT/A belegt und dessen physiologische Interaktion mit dem in der Plasmamembran vorhandenen SV2C inhibiert. Somit kann eine akute Intoxikation mit BoNT/A abgewendet werden. Weiterhin können diese Agenzien in kompetitiven Bindungsstudien zur Suche nach anderen Molekülen, die sich ebenfalls in die Rezeptorbindungsstelle im H_{C}-Fragment von BoNT/A einlagern und somit als Antagonisten wirken, eingesetzt werden. Durch eine Markierung der Agenzien z.B. mit Fluorophoren oder speziellen Erkennungssequenzen oder einer Immobilisierung an Feststoffphasen kann nach Bindung dieser Agenzien an BoNT/A jenes direkt spezifisch nachgewiesen werden. Damit ergibt sich die Möglichkeit, BoNT/A spezifisch in unterschiedlichen Umgebungen und Matrizes zu detektieren.

SV2C ist ein Glykoprotein aus Nervenzellen und neuroendokrinen Zellen (als Übersichtsartikel: Janz, R. und Südhof T.C., Neuroscience 94 (1999), 1279-1290). Es besteht aus 727 Aminosäuren mit einem Molekulargewicht von 86 kDa und ist mit 12 Transmembrandomänen in die Membran synaptischer Vesikel eingelagert. Der ca. 160 Aminosäuren lange Aminoterminus und der 11 Aminosäuren kurze Carboxylterminus befinden sich ebenso im Zytosol wie ein 90 Aminosäuren langer Abschnitt zwischen den Transmembrandomänen 6 und 7. Intravesikulär findet sich lediglich zwischen den Transmembrandomänen 7 und 8 ein 125 Aminosäuren langer Bereich (Aminosäuren 454-579), die intravesikuläre oder luminale Domäne (LD), welche drei putative N-Glykosylierungsstellen und zwei putative Disulfidbrücken enthält. Die Rolle von SV2C als Ionen- bzw. Zuckertransporter in synaptischen Vesikel hat sich nicht bestätigt, aber eine von der Phosphorylierung des Aminoterminus abhängige Interaktion der drei SV2 Isoformen mit denen des Synaptotagmins deutet daraufhin, dass über SV2 die Menge an freiem Synaptotagmin zur Bindung von Ca²⁺ und nachfolgender Initiation der Exozytose beeinflusst wird.

Durch Exozytose fusioniert die Membran der synaptischen Vesikel mit der präsynaptischen Plasmamembran, wodurch sich die synaptischen Vesikelproteine kurzzeitig auch in der präsynaptischen Membran befinden. Dadurch sind die intravesikulären Domänen der synaptischen Vesikelproteine extrazellulär exponiert. Die Bindung des H_{C}-Fragments von BoNT/A an die zahlreich vorkommenden komplexen Polysialoganglioside auf der Nervenzelloberfläche reicht zur Aufnahme des Neurotoxins allein nicht aus. Aber durch die Akkumulation der BoNT/A Moleküle auf der Nervenzelloberfläche können diese lateral in der Membran diffundieren, und die Wahrscheinlichkeit des produktiven Zusammentreffens mit dem selten exponierten Proteinrezeptor ist erhöht. Im Fall von SV2C wird die 125 Aminosäuren große luminale Domäne nach Vesikelfusion extrazellulär exponiert und steht somit für BoNT/A als Pröteinrezeptor zur Verfügung. Da sich das Neurotoxin aufgund der Gangliosidbindung recht dicht oberhalb der Membran aufhält, stehen analog zur BoNT/B/G-Synaptotagmin Interaktion bevorzugt die ca. 30 ersten und letzten Aminosäuren der luminalen Domäne als Rezeptor zur Verfügung. Nach Aufnahme des Rezeptor-Neurotoxin Komplexes in das Endosom wird dieses angesäuert, die Translokationsdomäne insertiert in die endosomale Membran und transloziert die partiell entfaltete LC ins Zytosol, wo diese im finalen Schritt ihr spezifisches Substrat spaltet. Der Zyklus der Komplexbildung und -dissoziation der Fusionsproteine wird unterbrochen und somit die Ausschüttung von Acetylcholin gehemmt. Als Folge hiervon werden gestreifte Muskeln gelähmt, und Schweißdrüsen stellen ihre Sekretion ein. Die Wirkdauer der einzelnen BoNT Serotypen ist verschieden und hängt von der Präsenz intakter LC im Zytosol ab.

Dass bevorzugt die cholinerge Transmission blockiert wird, kann damit erklärt werden, dass die HC in der Peripherie in das Neuron eindringt. Zentrale Synapsen werden durch die Blut-Him-Schranke geschützt, die von Proteinen nicht überwunden werden kann.

Nachfolgend werden Begriffe definiert, wie sie in Zusammenhang mit der vorliegenden Anmeldung zu verstehen sind.

Das rekombinant hergestellte Botulinus Neurotoxin A (BoNT/A) aus *E*. *coli,* welches u. a. die zum nativen Botulinus Neurotoxin A identische Aminosäuresequenz beinhaltet, verhält sich pharmakologisch identisch wie das native BoNT/A und wird rekombinantes Botulinus Neurotoxin Wildtyp genannt. Das rekombinant hergestellte H_{C}-Fragment von BoNT/A besitzt die gleiche Aminosäuresequenz wie das korrespondierende native H_{C}-Fragment und die gleichen Bindungseigenschaften wie das native BoNT/A. Die erwähnten Nervenzellen sind cholinerge Motoneurone. Vorzugsweise bindet das BoNT/A spezifisch an die Plasmamembran assoziierte Moleküle, Transmembranproteine, synaptische Vesikelproteine, ein Protein der SV2 Familie, vorzugsweise SV2C, besonders bevorzugt die luminale Domäne von SV2C. Die Bindung wird vorzugsweise *in vitro* bestimmt. Besonders bevorzugt erfolgt die Bestimmung durch Verwendung von GST-Pull-down Experimenten, die im Detail in den Beispielen ausgeführt sind.

Die Sequenz von SV2C ist für jedermann aus Datenbanken erhältlich. Die Sequenz ID für SV2C aus *Homo sapiens* lautet u. a. GenBank NP_055794, für SV2C aus *Rattus norvegicus* u. a. GenBank NP_113781, für SV2C aus *Mus musculus* u. a. GenBank XP_127490.

Der Ausdruck "Polypeptid" bedeutet in diesem Zusammenhang Aminosäurepolymere aus wenigstens zwei Monomereinheiten. Die Monomere können hierbei natürlich oder nicht natürlich vorkommende Aminosäuren sein. Bevorzugt weist ein Polypeptid wenigstens 10 Aminosäuremonomere auf. Die einzelnen Aminosäuren können hierbei modifiziert sein. Die Modifikationen können natürlichen Ursprungs sein (z. B. posttranslational) oder synthetisch eingeführt werden wie z.B. Glykosylierung, Di- und Oligomerisierung, und Modifikationen der Cys-Reste.

"% Identität" bedeutet erfindungsgemäß % Identität auf Protein-Ebene, die mittels bekannter Verfahren, z.B. der computergestützten Sequenzvergleiche (BLAST) Basic Local Alignment Search Tool, S.F. Altschul et al., J. Mol. Biol. 215 (1990), 403-410. Bevorzugte Verfahren zur Bestimmung der Identität erzeugen zunächst die größte Übereinstimmung zwischen den untersuchten Sequenzen. Sofern Sequenzpaare miteinander verglichen werden, können auch die Programme GAP (Devereux, J. et al., Nucleic Acids Res. 12812)= : 387 (1987) und BestFit verwendet werden. Generell ist die Verwendung von Standardparametern möglich, besonders bevorzugt sind diese:
Algorithmus: Needleman und Wunsch, J. Mol. Biol. 48:443-453(1970) Vergleichsmatrix: BLOSUM 62 aus Henikoff und Henikoff, PNAS USA 89(1992), 10915-10919
Lücken-Wert (Gap Penalty): 12
Lückenlängen-Wert (Gap Length Penalty): 4

Der Ausdruck Antikörper" schließt klassische Antikörper, single-chain-Antikörper und Antikörperfragmente ein. Bevorzugte Fragmente sind hierbei F(ab)2 und F(ab).

Der Ausdruck "Zusammensetzung" schließt neben Gemischen auch Fusionsproteine ein. Das in der Zusammensetzung enthaltene Polypeptid kann in Form eines Konjugates vorliegen. Hierbei sind Konjugate mit Farbstoffen, Eisenpartikeln, Epitopen wie Flag oder HA-Tag, Crosslinker, Affinitätspeptide oder radioaktive Isotope bevorzugt.

Gemäß einer weiteren bevorzugten Ausführungsform unterscheidet sich die Aminosäuresequenz des Polypeptides von der Aminosäuresequenz des synaptischen Vesikel Glykoproteins 2C aus H. sapiens durch die Addition, Substitution, Deletion, Insertion und/oder Inversion wenigstens einer Aminosäure, vorzugsweise nicht mehr als 5 Aminosäuren, insbesondere nicht mehr als 1 Aminosäure. Die Addition, Substitution, Deletion, Insertion oder Inversion kann hierbei in an sich bekannter Weise durchgeführt werden.

Die vorliegende Erfindung stellt ferner Nukleinsäuren bereit, die das erfindungsgemäß Polypeptid kodieren. Ferner werden erfindungsgemäß Vektoren bereitgestellt, die die erfindungsgemäße Nukleinsäure enthalten und zur Replikation und optional Expression unter der Kontrolle eines geeigneten Promotors in einer geeigneten Wirtszelle. Gemäß einem weiteren Aspekt der vorliegenden Erfindung wird ein Verfahren zur Herstellung des erfindungsgemäßen Polypeptides bereitgestellt, umfassend das rekombinante Exprimieren einer das Polypeptid kodierenden Nukleinsäure in einer geeigneten Wirtszelle und gegebenenfalls Isolieren des hergestellten Polypeptides in an sich bekannter Weise.

Die kodierende Nukleinsäure kann hierbei RNA, DNA oder Gemische davon darstellen. Die Nukleinsäure kann ferner im Hinblick auf ihre Nukleaseresistenz modifiziert sein wie z. B. durch Einfügung von Phosphorthioat-Bindungen. Die Nukleinsäure kann aus einer Ausgangs-Nukleinsäure hergestellt werden, wobei die Ausgangs-Nukleinsäure z. B. durch Klonierung aus genomischen oder cDNA-Banken zugänglich ist. Weiterhin kann die Nukleinsäure direkt durch Festphasensynthese hergestellt werden. Geeignete Verfahren sind dem Fachmann bekannt. Sofern von einer Ausgangs-Nukleinsäure ausgegangen wird, kann z. B. durch ortsgerichtete Mutagenese eine zielgerichtete Veränderung durchgeführt werden, die auf Aminosäure-Ebene zu wenigstens einer Addition, Insertion, Deletion und/oder Substitution führt. Die Nukleinsäure wird sodann in operativer Weise mit einem geeigneten Promotor verbunden. Geeignete Promotoren für die Expression in bekannten Expressionssystemen sind dem Fachmann bekannt. Die Wahl des Promotors hängt hierbei vom zur Expression verwendeten Expressionssystem ab. Generell sind konstitutive Promotoren bevorzugt, jedoch sind auch induzierbare Promotoren verwendbar. Das so hergestellte Konstrukt umfasst wenigstens einen Teil eines Vektors, insbesondere regulatorische Elemente, wobei der Vektor beispielsweise ausgewählt ist aus λ-Derivaten, Adenoviren, Baculoviren, Vacciniaviren, SV40-Viren und Retroviren. Der Vektor ist vorzugsweise zur Expression der Nukleinsäure in einer gegebenen Wirtszelle fähig.

Ferner stellt die Erfindung Wirtszellen bereit, die den Vektor enthalten und die zur Expression des Vektors geeignet sind. Im Stand der Technik sind zahlreiche prokaryontische und eukaryontische Expressionssysteme bekannt, wobei die Wirtszellen beispielsweise ausgewählt sind aus prokaryontischen Zellen wie *E. coli* oder *B. sublilis,* aus eukaryontischen Zellen wie *S. cerevisiae* und *P. pastoris* oder auch höheren eukaryontischen Zellen wie Insektenzellen oder Säugerzellen.

Das Peptid bzw. Polypeptid kann auch direkt durch Synthese oder Fragmentkondensation erhalten werden. Entsprechende Verfahren sind dem Fachmann bekannt.

Das Peptid bzw. Polypeptid wird nachfolgend aufgereinigt. Hierbei kommen dem Fachmann bekannte Verfahren zum Einsatz, wie z. B. Chromatographie-Verfahren oder Elektrophorese.

Gemäß einem weiteren Aspekt der vorliegenden Erfindung wird eine Zusammensetzung bereitgestellt, die wenigstens ein erfindungsgemäßes Polypeptid umfasst. Die Zusammensetzung kann hierbei als Gemisch oder als Konjugat vorliegen. Das Polypeptid kann beispielsweise mit Farbstoffmolekülen oder Trägern konjugiert sein.

Gemäß einem weiteren Aspekt der vorliegenden Erfindung wird ein Antikörper oder ein Fragment davon bereitgestellt, der/das an die Aminosäuresequenz, der luminalen Domäne (Aminosäuren 454-579) des synaptischen Vesikel Glykoproteins 2C aus *Homo sapiens,* bindet. Der Antikörper kann monoklonal oder polyklonal sein. Monoklonale Antikörper können gemäß dem Fachmann bekannten Verfahren durch Immunisieren von Versuchstieren wie Mäusen und nachfolgendem Isolieren und Screening von Hybridoma hergestellt werden.

Vorzugsweise ist der Antikörper fähig, die Bindung des synaptischen Vesikel Glykoproteins 2C an Botulinus Neurotoxin zu blockieren. Dies kann in bekannten kompetitiven Assays wie Radioimmunoassays oder ELISAs nachgewiesen werden.

Gemäß einem weiteren Aspekt der vorliegenden Erfindung wird eine pharmazeutische Zusammensetzung bereitgestellt, die wenigstens ein erfindungsgemäßes Polypeptid und/oder wenigstens einen erfindungsgemäßen Antikörper umfasst. Die pharmazeutische Zusammensetzung kann optional einen pharmazeutisch akzeptablen Träger, ein Verdünnungsmittel und/oder Additiv enthalten. Die pharmazeutische Zusammensetzung ist zur oralen, intravenösen, subkutanen, intramuskulären und topischen Verabreichung geeignet.

Die pharmazeutische Zusammensetzung wird zur Behandlung bei Botulismus, nach Überdosierung bei therapeutischer Behandlung oder kosmetischer Anwendung von BoNT/A, Intoxikation oder zur Prophylaxe angegeben.

Gemäß einem weiteren Aspekt der vorliegenden Erfindung wird ein Verfahren zur Reduktion der Neurotoxizität von BoNT/A in Säugetieren bereitgestellt, umfassend das Verabreichen eines Agens an das Säugetier, wobei das Agens die Bindung von BoNT/A an der luminalen Domäne (Aminosäuren 454-579) des synaptischen Vesikel Glykoproteins 2C aus *Homo sapiens* um wenigstens 10 % vermindert, vorzugsweise um wenigstens 50 %, insbesondere um wenigstens 80 % vermindert.

Das Agens kann hierbei durch das nachstehend beschriebene Screening-Verfahren bestimmt werden.

Bevorzugt wird dem Säugetier ein Polypeptid gegeben, welches identische Aminosäuresequenz zur luminalen Domäne (Aminosäuren 454-579) des synaptischen Vesikel Glykoproteins 2C (SV2C) aus *Homo sapiens* aufweist und welches die Bindung von BoNT/A an SV2C vermindert.

Weiterhin bevorzugt wird dem Säugetier ein Antikörper gegeben wird, welcher die Bindung von BoNT/A an der luminalen Domäne (Aminosäuren 454-579) des synaptischen Vesikel Glykoproteins 2C aus *Homo sapiens* vermindert.

Weiterhin bevorzugt wird dem Säugetier ein Agens gegeben wird, welches die Expression der luminalen Domäne (Aminosäuren 454-579) des synaptischen Vesikel Glykoproteins 2C aus *Homo sapiens* vermindert. Das Agens kann hierbei ein antisense-Molekül sein. Verfahren zur Herstellung von antisense-Molekülen sind dem Fachmann bekannt.

Das Verfahren kann zur Reduktion der Neurotoxizität von BoNT/A bei Botulismus, nach Überdosierung bei therapeutischer Behandlung oder kosmetischer Anwendung von BoNT/A, Intoxikation oder zur Prophylaxe verwendet werden.

Vorzugsweise ist das Säugetier *Homo sapiens.*

Gemäß einem weiteren Aspekt der vorliegenden Erfindung wird ein Verfahren zur Identifizierung eines Agens bereitgestellt, welches die Bindung von BoNT/A an die luminale Domäne (Aminosäuren 454-579) des synaptischen Vesikel Glykoproteins 2C aus *Homo sapiens* vermindert, umfassend:
(a) das Inkontaktbringen eines Agens mit einer Lösung aus BoNT/A und GST-SV2C (454-579)
(b) Bestimmen der Menge an gebundenem GST-SV2C(454-579)
(c) Auswählen des Agens, das die an GST-SV2C (454-579) gebundene Menge von BoNT/A verringert.

Das Screening kann hierbei z.B. mit chemischen Bibliotheken (chemical libraries) durchgeführt werden. Weiterhin werden auch Banken aus DNA und/oder RNA-Molekülen in Erwägung gezogen.

Vorzugsweise ist die luminale Domäne (Aminosäuren 454-579) des synaptischen Vesikel Glykoproteins 2C aus *Homo sapiens* in eine Plasmamembran einer Zelle eingelagert. Hierbei sind geeignete Zellen neuroendokrine Zellen wie PC 12, Neuroblastoma-Zellen wie 2A, und Hybridoma-Zellen aus embryonalem Hirngewebe wie NT2.

Weiterhin bevorzugt wird die durch die Gegenwart des Agens verminderte Bindung von BoNT/A an das synaptische Vesikel Glykoproteins 2C aus *Homo sapiens* nachgewiesen durch eine verringerte Neurotoxizität des BoNT/A im Maus Hemidiaphragma Test.

Gemäß einem weiteren Aspekt der vorliegenden Erfindung wird das durch das vorstehend beschriebene Verfahren erhältliche Agens bereitgestellt.

Gemäß einem weiteren Aspekt der vorliegenden Erfindung wird ein Verfahren zum Nachweis von BoNT/A aus *Clostridium botulinum* in einer beliebigen Probe bereitgestellt, umfassend:
(a) Immobilisieren eines Polypeptides an einer Festphase, wobei das Polypeptid eine mindestens zu 70% identische Aminosäuresequenz zur luminalen Domäne (Aminosäuren 454-579) des synaptischen Vesikel Glykoproteins 2C aus *Homo sapiens* aufweist und welches die Bindung von BoNT/A an SV2C vermindert;
(b) Inkontaktbringen des immobilisierten Polypeptides mit einer Probe unter Bedingungen, die Bindung von BoNT/A an das Polypeptid erlauben;
(c) Eluieren des BoNT/A-Polypeptid-Komplexes; und
(d) Nachweisen des Komplexes oder seiner Bestandteile.

Das Immobilisieren kann beispielsweise durch BrCN-Kopplung des Polypeptides an für Chromatographiezwecke bekannte Materialien wie Sepharose erfolgen. Die Bedingungen, die die Bindung von BoNT/A an das Polypeptid erfolgen können durch Routine-Versuche bestimmt werden. Die Bedingungen sind so zu wählen, dass keiner der Bindungspartner denaturiert. Das Eluieren kann beispielsweise durch Kompetition oder Veränderung der pH- und/oder Salzbedingungen erfolgen. Der Komplex oder seine Bestandteile können beispielsweise durch SDS-PAGE aufgetrennt werden.

Das folgende Beispiel dient lediglich dem Zwecke der Verdeutlichung und sollte nicht als begrenzend angesehen werden.

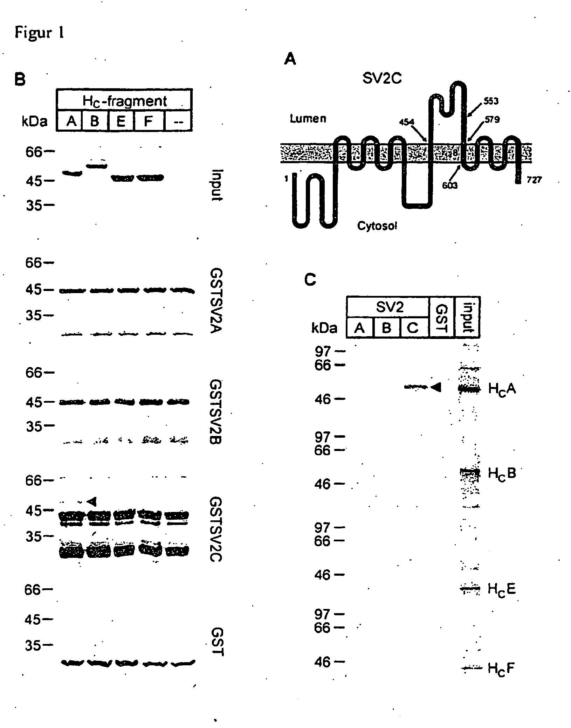

- Figur 1:: Das H_{C}-Fragment von BoNT/A interagiert mit SV2C. (A) Schematische Darstellung der synaptischen Vesikel Glykoproteine. (B, C) GST-Fusionproteine immobilisiert an GT-Sepharosekügelchen, werden mit rekombinanten (B) or ³⁵S-markierten (C) BoNT H_{C}-Fragmenten in der Gegenwart von Gangliosiden inkubiert. An der Festphase gebundene H_{C}-Fragmente werden durch SDS-PAGE und Coomassie Blau Färbung oder Autoradiography nachgewiesen.

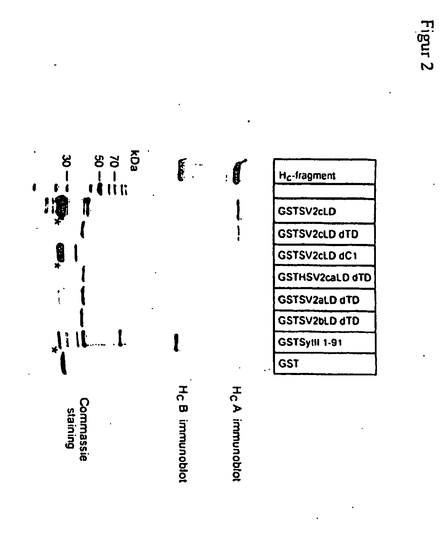

- Figur 2:: BoNT/A H_{C}-Fragment bindet an die intravesiculäre Region benachbart zur Transmembrandomäne 8 von SV2C. GST-Fusionproteine immobilisiert an GT-Sepharosekügelchen, werden mit rekombinanten BoNT H_{C}-Fragmenten in der Gegenwart von Gangliosiden inkubiert. An der Festphase gebundene H_{C}-Fragmente werden durch SDS-PAGE und Coomassie Blau Färbung oder immunchemisch nachgewiesen.

### Material und Methoden

### Plasmidkonstruktion und Herstellung rekombinanter Proteine

Plasmide für die *E*. *coli* Expression von volle Länge BoNT/A und B bzw. rekombinanten H_{c}-Fragmenten von BoNT/A-G und TeNT mit carboxyl-terminalen StrepTag zur Affinitätsaufreinigung bzw. zur *in vitro* Transkription/Translation mit ³⁵S-Methionin wurden durch PCR-Verfahren mit geeigneten Primern, BoNT/A-G und TeNT kodierender cDNA und dem Expressionsvektor pQe3 (Qiagen AG) bzw. pSP72 (Promega) als Ausgangsvektor erzeugt.

cDNA Abschnitte, die die intravesikulären Segmente der verschiedenen synaptischen Vesikel Protein kodieren, wurden in den Vektor pGEX-4T3 unter Benutzung entsprechender Oligonukleotide und einer embryonalen Maus cDNA-Bank (GST-Syo-157-218, GST-Syg-I-45-98) oder von Plasmiden mit entsprechender cDNA als Vorlage kloniert: GST-Syg-I-127-169 (RZPD, Deutsches Ressourcenzentrum für Genomforschung GmbH; www.rzpd.de; ID IRAKp961C072Q), GST-SV2A-468-618 (RZPD-ID IRAKp961024100Q), GST-Syo-22-103 (Ratte; T. C. Südhof, Dallas), GST-Syg-III-46-88 und GST-Syg-III-128-168 (Maus; T. C. Südhof, Dallas), GST-SV2B-413-560 (Ratte; S. Bajjalieh, Seattle), und GST-SV2C-454-603 (Ratte; R. Janz, Houston). Ein das Hybrid GST-SV2-C/A kodierende Plasmid beinhaltet die Aminosäuren 454-553 von SV2C und 568-594 von SV2A und wurde ebenfalls mit den entsprechenden Oligonukleotiden per PCR generiert. Die Nukleinsäuresequenzen sämtlicher Plasmide wurden durch DNA-Sequenzierung bestätigt. Die rekombinanten H_{c}-Fragmente wurden im *E*. *coli-*Stamm M 15 [pRep4] (Qiagen) während einer zehnstündigen Induktion bei Raumtemperatur hergestellt und an einer StrepTactin-Matrix (IBA GmbH) gemäß den Herstellerempfehlungen aufgereinigt. Die aus *E*. *coli* BL21 erhaltenen GST-Fusionsproteine wurden mit Hilfe von auf Sepharosekügelchen immobilisierten Glutathion isoliert. Fraktionen, die die gewünschten Proteine enthielten, wurden vereinigt und gegen Tris-NaCl-Triton-Puffer (20 mM Tris-HCl, 150 mM NaCl, 0,5 % Triton X-100, pH 7,2) bzw. Kreb-Ringer Puffer (118 mM NaCl, 4.7 mM KCI, 1.2 mM MgSO₄, 1.2 mM KH₂PO₄, 25 mM NaHCO₃, 2.5 mM CaCl₂, 11 mM Glukose) dialysiert.

³⁵S-markierte H_{c}-Fragmente wurden *in vitro* aus pSP72 Derivaten, die stromabwärts vom carboxyl-terminalen Kodon des Neurotoxins linearisiert wurden, unter Benutzung des Reticulozytenlysatsystems. (Promega, Mannheim) und L-³⁵S-Methionin (840 kBq, >37 TBq/mmol; Amersham Biosciences) in einem Ansatz von 2 µl synthetisiert

### GST-Pull-down Assav

GST-Fusionsproteine (jeweils 0,15 nmol), die auf 10 µl GT-Sepharose-Kügel chen immobilisiert waren, wurden mit H_{c}-Fragmenten (0,1 nmol) in Abwesenheit oder Gegenwart eines Rinderhirn-Gangliosid-Gemisches (18% GM1, 55 % GD1a, 10 % GT1b, 2 % sonstige. Gangloside; Calbiochem; 20 µg jeweils) in einem Gesamtvolumen von 100 µl Tris-NaCl-Triton-Puffer für 3 h bei 4 °C inkubiert. Die Kügelchen wurden durch Zentrifugation gesammelt, der Überstand entfernt und die separierten Kügelchen jeweils dreimal mit 160 µl des gleichen Puffers gewaschen. Die gewaschenen Pellet-Fraktionen wurden in SDS-Probenpuffer aufgekocht und zusammen mit den Überstandfraktionen durch SDS-PAGE und Coomassie Blaufärbung, Autoradiography oder Immunoblotting untersucht.

### Maus Hemidiaphragma Assay (HDA)

Der Maus Hemidiaphragma Assay (HDA) wurde durchgeführt wie unter Habermann (Habermann E, Dreyer F, Bigalke H (1980) Tetanus toxin blocks the neuromuscular transmission in vitro like botulinum A toxin. Naunyn Schmiedebergs Arch Pharmacol 311: 33-40) beschrieben. Der Nervus phrenicus wurde mit einem Hertz stimuliert und die Kontraktionsamplitude kontinuierlich mittels Kraftmesser und der VitroDat Online Software (FMI GmbH, Seeheim-Ober Beerbach,) aufgezeichnet. Nach Zugabe des BoNTs wurde die Zeit gemessen, in der die Kontraktionsamplitude auf 50 % des Ausgangwertes abfällt (Paralytische Halbzeit). Volle Länge scBoNT/A Wildtyp wurde mindestens dreifach in folgenden Endkonzentrationen gemessen: 24,3 pM, 72,8 pM, 223 pM, und 728 pM. An diese Konzentrations-Wirkungsbeziehung wurde eine Potenzfunktion angenähert:

y(A) = 225,87x^{-0,2573} (R²= 0,9627). In gleicher Weise wurde die Konzentrations-Wirkungsbeziehung y(B) = 423,59x^{-0.297} (R² = 0,983) für volle Länge scBoNT/B Wildtyp mit folgenden Endkonzentrationen erstellt: 100 pM, 300 pM, 1000 pM, und 3000 pM. Für die Inhibitionstudien wurden scBoNT/A (223 pM) und scBoNT/B (1000 pM) entweder mit GST-SV2C-454-579 oder GST-SV2-C/A (Endkonzentration 4,7 oder 11,2 µM) gemischt, 15 min bei 20°C inkubiert und dem HDA zugefügt. Anhand der Potenzfunktionen wurden die z. T. verlängerten paralytischen Halbzeiten in entsprechend niedrigere Neurotoxindosen umgerechnet und als % Toxizität ausgedrückt.

### Ergebnisse

Die luminalen Domänen und die carboxyl-terminalen Transmembrandomänen der Proteine Synaptophysin, Synaptoporin, Synaptogyrin 1 & III, Synaptotagmin II, SV2A, SV2B und SV2C wurden subkloniert und als Gluthathion-S-Transferase-(GST)-Fusionsprotein rekombinant in *E. coli* exprimiert und isoliert. Die H_{c}-Fragmente der sieben BoNTs und von Tetanus Neurotoxin (TeNT) wurden sowohl rekombinant in *E. coli* exprimiert als auch mit ³⁵S-Methionin *in vitro* translatiert. Die Affinität der H_{C}-Fragmente an die luminalen Domänen der oben aufgeführten GST-Fusionsproteine wurde in Gluthathion-S-Transferase-(GST)-Pull-down Experimenten bestimmt. Hierzu wurde das jeweilige GST-Fusionsprotein mit unterschiedlichen H_{C}-Fragmenten inkubiert und eine Phasentrennung durchgeführt. Freies H_{C}-Fragment verblieb im separierten Überstand, während gebundenes BoNT H_{C}-Fragment in der Festphase zusammen mit dem GST-Fusionsprotein nachweisbar war. Der Ersatz der rekombinanten H_{C}-Fragmente durch ³⁵S-markierte H_{C}-Fragmente sowie von volle Länge BoNT/A ergab im GST-Pull-down Experimenten im Vergleich zum H_{C}-Fragment von BoNT/A dieselben Ergebnisse.

**Tabelle 1: Die intravesikuläre Domain von SV2C blockiert die Aktivität von BoNT/A im HDA.**

| BoNT | BoNT [pM] | Inhibitor^{a} | Inhibitor [µM] | Paral. Halbwenszeit t_{1/2}^{b} [min] | Toxizität *versus* reinem BoNT [%] | Inhibilion [%] |
|---|---|---|---|---|---|---|
| BoNT/A | 223 | kein | | 50 ± 7 | 100 | |
| BoNT/A | 223 | SV2C-454-579 | 4,7 | 72 ± 3 | 25,1 ± 1,0 | 75 |
| BoNT/A | 223 | SV2C-454-579 | 11,2 | 83 ± 8 | 14,6 ± 1,5 | 85 |
| BoNT/A | 1000 | kein | | 57 ± 6 | 100 | |
| BoNT/A | 1000 | SV2C-454-579 | 4,7 | 56 ± 13 | 105,1 ± 24,8 | 0 |
| BoNT/A | 1000 | SV2C-454-579 | 11,2 | 60 ± 7 | 86,6 ± 9,7 | 14 |
| BoNT/A | 223 | SV2-C/A | 4,7 | 51 ± 4 | 100,0 ± 1,0 | 0 |
| BoNT/A | 223 | SV2-C/A | 11.2 | 51 ± 5 | 100,0 ± 1,0 | 0 |

| | | | | | | |
|---|---|---|---|---|---|---|
| ^{a} Inhibitoren wurden als GST-Fusionsproteine eingesetzt ^{b} Mittelwerte ± S.D. (n = 3-9) ^{c} Volle Länge scBoNT/A Wildtyp wurde mindestens dreifach in folgenden Endkonzentrationen gemessen: 24,3 pM, 72,8 pM, 223 pM, und 728 pM. An diese Konzentrations-Wirkungsbeziehung wurde eine Potenzfunktion angenähert: y(A) = 225,87x^{-0.22573} (R² = 0,9627). In gleicher Weise wurde die Konzentrations-Wirkungsbeziehung y(B) = 423,59x^{-0.297} (R² = 0,983) für volle Länge scBoNT/B Wildtyp mit folgenden Endkonzentrationen erstellt: 100 pM, 300 pM, 1000 pM, und 3000 pM. | | | | | | |

Dabei wurde herausgefunden, dass keines der acht H_{c}-Fragmente von BoNT/A, B, Cl, D, E, F, G und TeNT an die luminalen Domänen von Synaptophysin, Synaptoporin, Synaptogyrin I & III, SV2A und SV2B unabhängig von der Anwesenheit von komplexen Gangliosiden bindet. Wie bereits bekannt binden die H_{c}-Fragmente von BoNT/B und G an die luminale Domäne von Synaptotagmin II, nicht aber das H_{c}-Fragment von BoNT/A. Einzig das rekombinante als auch das ³⁵S-markierte H_{c}-Fragnent sowie das volle Länge BoNT/A binden spezifisch an die an GST fusionierte luminale Domäne von SV2C unabhängig von der Anwesenheit von komplexen Gangliosiden. Alle anderen H_{c}-Fragmente zeigen keine Interaktion mit SV2C (Figur 1).

Weiterhin wurde gezeigt, dass die Bindung des H_{c}-Fragments von BoNT/A an die luminale Domäne von SV2C nach Verkürzung um die Transmembrandomäne 8 (GST-SV2C 454-579) schwächer ist (Figur 2). Eine carboxyl-terminale Deletion um 20 Aminosäuren (GST-SV2C 454-553) und weitere Verkürzungen führten zum Erliegen der Wechselwirkung mit BoNT/A. Auch amino-terminale Deletionen verhinderten die Bindung von BoNT/A an die GST-SV2C Fusionsproteine.

Die GST-Fusionsproteine der zu SV2C homologen SV2A und SV2B zeigten weder mit noch ohne carboxyl-terminaler Transmembrandomäne Bindung an BoNT/A. Die Generation eines Hybrids bestehend aus GST, den Aminosäuren 454-554 von SV2C und den Aminosäuren 568-594 von SV2A wies ebenfalls keinerlei Interaktion mit BoNT/A H_{c}-Fragment mehr auf.

## Patentansprüche

1. Isoliertes Polypeptid der luminalen Domäne (Aminosäuren 454-579) des synaptischen Vesikel Glykoproteins 2C aus *Homo sapiens,* wobei das isolierte Polypeptid das H_{c}-Fragment von Botulinus Neurotoxin A bindet.

2. Polypeptid gemäß Anspruch 1, wobei die Aminosäuresequenz des Polypeptides sich von der Aminosäuresequenz der luminalen Domäne des synaptischen Vesikel Glykoproteins 2C aus H. sapiens durch die Addition, Substitution, Deletion, Insertion und/oder Inversion wenigstens einer Aminosäure, aber nicht mehr als 5 Aminosäuren, insbesondere nicht mehr als 1 Aminosäure unterscheidet.

3. Nukleinsäure kodierend ein Polypeptid gemäß einem der Ansprüche 1 bis 2.

4. Vektor enthaltend eine Nukleinsäure gemäß Anspruch 3 und ferner einen zur Expressionskontrolle geeigneten Promotor, wobei die für das Polypeptid kodierende Nukleinsäure unter der Kontrolle des Promotors steht.

5. Wirtszelle enthaltend eine Nukleinsäure gemäß Anspruch 3 und/oder einen Vektor gemäß Anspruch 4.

6. Verfahren zur Herstellung des Polypeptides nach einem der Ansprüche 1 bis 2 umfassend das rekombinante Exprimieren einer das Polypeptid kodierenden Nukleinsäure gemäß Anspruch 3 und/oder eines Vektors gemäß Anspruch 4 in einer geeigneten Wirtszelle und gegebenenfalls Isolieren des hergestellten Polypeptides in an sich bekannter Weise.

7. Zusammensetzung umfassend wenigstens ein Polypeptid gemäß einem der Ansprüche 1 bis 2.

8. Antikörper zur Reduktion der Neurotoxizität von BoNT/A in Säugetieren, welcher die Bindung von BoNT/A an die Aminosäuresequenz der lumina-len Domäne (Aminosäuren 454-579) des synaptischen Vesikel Glykopro-teins 2C aus Homo sapiens um wenigstens 10 % vermindert, vorzugsweise um wenigstens 50 %, insbesondere um wenigstens 80 % vermindert, wobei der Antikörper an die Aminosäuresequenz der luminalen Domäne (Aminosäuren 454-579) des SV2C aus Homo sapiens bindet.

9. Antikörper nach Anspruch 8, zur Reduktion der Neurotoxizität von BoNT/A bei Botulismus, nach Überdosierung bei therapeutischer Behandlung oder kosmetischer Anwendung von BoNT/A, Intoxikation oder zur Prophylaxe.

10. Antikörper nach einem der Ansprüche 8 bis 9, wobei das Säugetier *Homo sapiens* ist.

11. Antisense-Molekül zur Reduktion der Neurotoxizität von BoNT/A in Säugetieren, welches die Expression der luminalen Domäne (Aminosäuren 454-579) des synaptischen Vesikel Glykoproteins 2C aus *Homo sapiens* vermindert.

12. Antisense-Molekül nach Anspruch 11, zur Reduktion der Neurotoxizität von BoNT/A bei Botulismus, nach Überdosierung bei therapeutischer Behandlung oder kosmetischer Anwendung von BoNT/A, Intoxikation oder zur Prophylaxe.

13. Antisense-Molekül nach einem der Ansprüche 11 bis 12, wobei das Säugetier *Homo sapiens* ist.

14. Verfahren zur Identifizierung eines Antikörpers oder antisense-Moleküls, welcher/welches die Bindung von BoNT/A an die luminale Domäne (Aminosäuren 454-579) des synaptischen Vesikel Glykoproteins 2C aus *Homo sapi*ens vermindert, umfassend:
(a) das Inkontaktbringen eines Antikörpers oder antisense-Moleküls mit einer Lösung aus BoNT/A und GST-SV2C (454-579)
(b) Bestimmen der Menge an gebundenem GST-SV2C(454-579)
(c) Auswählen des Antikörpers oder antisense-Moleküls, das die an GST-SV2C (454-579) gebundene Menge an BoNT/A verringert.

15. Verfahren nach Anspruch 14, wobei die luminale Domäne (Aminosäuren 454-579) des synaptischen Vesikel Glykoproteins 2C aus *Homo sapiens* in eine Plasmamembran einer Zelle eingelagert ist.

16. Verfahren nach Anspruch 14 oder 15, wobei die durch die Gegenwart des Antikörpers oder antisense-Moleküls verminderte Bindung von BoNT/A an das synaptische Vesikel Glykoproteins 2C aus *Homo sapiens* nachgewiesen wird durch eine verringerte Neurotoxizität des BoNT/A im Maus Hemidiaphragma Test.

17. Pharmazeutische Zusammensetzung umfassend wenigstens ein Polypeptid gemäß einem der Ansprüche 1 bis 2 und/oder wenigstens einen Antikörper, wobei der Antikörper an die Aminosäuresequenz der luminalen Domäne (454-579) des synaptischen Vesikel Glykoproteins 2C aus *Homo sapiens* bindet.

18. Verfahren zum Nachweis von BoNT/A aus *Clostridium botulinum* in einer beliebigen Probe, umfassend:
(a) Immobilisieren eines Polypeptides an einer Festphase, wobei das Polypeptid eine mindestens zu 70% identische Aminosäuresequenz zur luminalen Domäne (Aminosäuren 454-579) des synaptischen Vesikel Glykoproteins 2C aus *Homo sapiens* aufweist und welches die Bindung von BoNT/A an SV2C vermindert;
(b) Inkontaktbringen des immobilisierten Polypeptides mit einer Probe unter Bedingungen, die Bindung von BoNT/A an das Polypeptid erlauben;
(c) Eluieren des BoNT/A-Polypeptid-Komplexes; und
(d) Nachweisen des Komplexes oder seiner Bestandteile.

## Claims

1. An isolated polypeptide of the luminal domain (amino acids 454-579) of the synaptic vesicle glycoprotein 2C of *Homo sapiens,* wherein the isolated polypeptide binds the H_{C}-fragment of botulinum neurotoxin A.

2. The polypeptide according to claim 1, wherein the amino acid sequence of the polypeptide differs from the amino acid sequence of the synaptic vesicle glycoprotein 2C of *Homo sapiens* by the addition, substitution, deletion, insertion and/or inversion of at least one amino acid, however not more than 5 amino acids, in particular not more than 1 amino acid.

3. A nucleic acid coding for a polypeptide according to anyone of claims 1 to 2.

4. A vector containing a nucleic acid according to claim 3 and further containing a promoter suitable for expression control, wherein the nucleic acid coding for the polypeptide is controlled by the promoter.

5. A host cell containing a nucleic acid according to claim 3 and/or a vector according to claim 4.

6. A process for the preparation of the polypeptide according to anyone of claims 1 to 2, comprising the recombinant expression of a nucleic acid coding for the polypeptide according to claim 3 and/or a vector according to claim 4 in an appropriate host cell and, as the case may be, isolating the produced polypeptide in a *per* se known manner.

7. A composition comprising at least one polypeptide according to anyone of claims 1 to 2.

8. An antibody for reducing the neurotoxicity of BoNT/A in mammals, which reduces binding of BoNT/A to the amino acid sequence of the luminal domain (amino acids 454-579) of the synaptic vesicle glycoproteins 2C of *Homo* sapiens by at least 10 %, preferably by at least 50 %, in particular by at least 80 %, wherein the antibody binds to the amino acid sequence of the luminal domain (amino acids 454-579) of SV2C of *Homo sapiens.*

9. The antibody according to claim 8, for reducing the neurotoxicity of BoNT/A in botulism, after an overdose during therapeutic treatment or cosmetic application of BoNT/A, intoxication or for prophylactic purposes.

10. Antibody according to any of claims 8 to 9, wherein the mammal is *Homo sapiens.*

11. An antisense molecule for reducing the neurotoxicity of BoNT/A in mammals, reducing the expression of the luminal domain (amino acids 454-579) of the synaptic vesicle glycoprotein 2C of *Homo sapiens.*

12. The antisense molecule according to claim 11, for reducing the neurotoxicity of BoNT/A in botulism, after an overdose during therapeutic treatment or cosmetic application of BoNT/A, intoxication or for prophylactic purposes.

13. The antisense molecule according to any of claims 11 to 12, wherein the mammal is *Homo sapiens.*

14. A process for identifying an antibody or antisense molecule that reduces binding of BoNT/A to the luminal domain (amino acids 454-579) of the synaptic vesicle glycoprotein 2C of *Homo sapiens,* comprising:
(a) bringing into contact an antibody or antisense molecule with a solution of BoNT/A and GST-SV2C (454-579)
(b) determining the quantity of bound GST-SV2C (454-579)
(c) selecting the antibody or antisense molecule, which reduces the quantity of BoNT/A bound to GST-SV2C (454-579).

15. The process according to claim 14, wherein the luminal domain (amino acids 454-579) of the synaptic vesicle glycoprotein 2C of *Homo sapiens* is embedded in a plasma membrane of a cell.

16. The process according to claims 14 or 15, wherein binding of BoNT/A to the synaptic vesicle glycoprotein 2C of *Homo sapiens,* reduced by the presence of the antibody or antisense molecule, is detected by decreased neurotoxicity of the BoNT/A in the mouse hemidiaphragma test.

17. A pharmaceutical composition comprising at least one polypeptide according to any one of claims 1 to 2 and/or at least one antibody, wherein the antibody binds to the amino acid sequence of the luminal domain (454-579) of the synaptic vesicle glycoprotein 2C of *Homo sapiens.*

18. A process for detecting BoNT/A from *Clostridium botulinum* in any desired sample, comprising:
(a) immobilizing a polypeptide on a solid phase, wherein the polypeptide has an amino acid sequence, at least 70 % of which is identical to the luminal domain (amino acids 454-579) of the synaptic vesicle glycoprotein 2C *of Homo sapiens,* and which reduces binding of BoNT/A to SV2C;
(b) bringing into contact the immobilized polypeptide with a sample under conditions permitting binding of BoNT/A to the polypeptide;
(c) eluting the BoNT/A polypeptide complex; and
(d) detecting the complex or its elements.

## Revendications

1. Polypeptide isolé des domaines luminaux (acides aminés 454-579) de la glycoprotéine 2C des vésicules synaptiques de *Homo sapiens,* le polypeptide isolé liant le fragment H_{c} de la neurotoxine botulique A.

2. Polypeptide selon la revendication 1, dans lequel la séquence d'acides aminés du polypeptide diffère de la séquence d'acides aminés des domaines luminaux de la glycoprotéine 2C des vésicules synaptiques de *H*. *sapiens* par addition, substitution, délétion, insertion et/ou inversion d'au moins un acide aminé, mais de pas plus de 5 acides aminés, en particulier de pas plus de 1 acide aminé.

3. Acide nucléique codant pour un polypeptide selon l'une des revendications 1 à 2.

4. Vecteur contenant un acide nucléique selon la revendication 3, et en outre un promoteur convenant au contrôle de l'expression, l'acide nucléique codant pour le polypeptide étant sous le contrôle du promoteur.

5. Cellule hôte contenant un acide nucléique selon la revendication 3 et/ou un vecteur selon la revendication 4.

6. Procédé de préparation d'un polypeptide selon l'une des revendications 1 à 2, comprenant l'expression par recombinaison d'un acide nucléique codant pour un polypeptide selon la revendication 3 et/ou d'un vecteur selon la revendication 4 dans une cellule hôte appropriée, et éventuellement l'isolement, d'une manière connue en soi, du polypeptide préparé.

7. Composition comprenant au moins un polypeptide selon l'une des revendications 1 à 2.

8. Anticorps pour la réduction de la neurotoxicité de la BoNT/A chez les mammifères, qui diminue la liaison de la BoNT/A à la séquence d'acides aminés des domaines luminaux (acides aminés 454-579) de la glycoprotéine 2C des vésicules synaptiques de *Homo sapiens,* d'au moins 10 %, de préférence d'au moins 50 %, en particulier d'au moins 80 %, l'anticorps se liant à la séquence d'acides aminés des domaines luminaux (acides aminés 454-579) du SV2C de *Homo sapiens.*

9. Anticorps selon la revendication 8, pour la réduction de la neurotoxicité de la BoNT/A en présence d'un botulisme, après surdosage dans le cadre du traitement thérapeutique ou de l'utilisation cosmétique de la BoNT/A, après intoxication, ou pour la prophylaxie.

10. Anticorps selon l'une des revendications 8 à 9, pour lequel le mammifère est *Homo sapiens.*

11. Molécule antisens pour la réduction de la neurotoxicité de la BoNT/A chez les mammifères, qui diminue l'expression des domaines luminaux (acides aminés 454-579) de la glycoprotéine 2C des vésicules synaptiques de *Homo sapiens.*

12. Molécule antisens selon la revendication 1, pour la réduction de la neurotoxicité de la BoNT/A en présence d'un botulisme, après surdosage dans le cadre d'un traitement thérapeutique ou d'une utilisation cosmétique de la BoNT/A, d'une intoxication, ou pour la prophylaxie.

13. Molécule antisens selon l'une des revendications 11 à 12, pour laquelle le mammifère est *Homo sapiens.*

14. Procédé pour identifier un anticorps ou une molécule antisens, qui diminue la liaison de la BoNT/A aux domaines luminaux (acides aminés 454-579) de la glycoprotéine 2C des vésicules synaptiques de *Homo sapiens*, comprenant :
(a) la mise en contact d'un anticorps ou d'une molécule antisens avec une solution de BoNT/A et de GST-SV2C (454-579),
(b) la détermination de la quantité de GST-SV2C (454-579) lié,
(c) la sélection de l'anticorps ou de la molécule antisens qui diminue la quantité de BoNT/A liée au GST-SV2C (454-579).

15. Procédé selon la revendication 14, dans lequel les domaines luminaux (acides aminés 454-579) de la glycoprotéine 2C des vésicules synaptiques de *Homo sapiens* sont incorporés dans une membrane plasmatique d'une cellule.

16. Procédé selon la revendication 14 ou 15, dans lequel la liaison, diminuée par la présence de l'anticorps ou de la molécule antisens, de la BoNT/A à la glycoprotéine 2C de vésicules synaptiques de *Homo sapiens,* est détectée par une diminution de la neurotoxicité de la BoNT/A dans le test de l'hémidiaphragme de souris.

17. Composition pharmaceutique comprenant au moins un polypeptide selon l'une des revendications 1 à 2 et/ou au moins un anticorps, l'anticorps se liant à la séquence d'acides aminés des domaines luminaux (454-579) de la glycoprotéine 2C des vésicules synaptiques de *Homo sapiens.*

18. Procédé de détection de la BoNT/A de *Clostridum botulinum* dans un échantillon quelconque, comprenant :
(a) l'immobilisation d'un polypeptide à une phase solide, le polypeptide ayant une séquence d'acides aminés présentant une identité d'au moins 70 % avec les domaines luminaux (acides aminés 454-579) de la glycoprotéine 2C des vésicules synaptiques de *Homo sapiens,* et qui diminue la liaison de la BoNT/A au SV2C ;
(b) la mise en contact du polypeptide immobilisé avec un échantillon dans des conditions qui permettent la liaison de la BoNT/A au polypeptide ;
(c) la élution du complexe BoNT/A-polypeptide ; et
(d) la détection du complexe ou de ses constituants.
